(19) Europäisches Patentamt
European Patent Office
Office européen des brevets

(11) **EP 3 127 500 B1**

(12) **EUROPEAN PATENT SPECIFICATION**

(45) Date of publication and mention
of the grant of the patent:
**12.06.2019   Bulletin 2019/24**

(51) Int Cl.:
***A61B 17/82*** *(2006.01)*      ***A61B 17/84*** *(2006.01)*

(21) Application number: **16182892.6**

(22) Date of filing: **04.08.2016**

(54) **INTERNAL BONE FIXATION DEVICE**

VORRICHTUNG ZUR INTERNEN KNOCHENFIXIERUNG

DISPOSITIF DE FIXATION D'OS INTERNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.08.2015   TW 104125361**

(43) Date of publication of application:
**08.02.2017   Bulletin 2017/06**

(73) Proprietor: **Human Origin Biotechnology Co., Ltd.
New Taipei City 242 (TW)**

(72) Inventors:
• **Dai, Lien-Guo**
**406 Taichung (TW)**
• **Chang, Ya-Hui**
**406 Taichung (TW)**
• **Dai, Shiuan-Yuan**
**406 Taichung (TW)**
• **Dai, Shiuan-Te**
**406 Taichung (TW)**
• **Dai, Shiuan-Jen**
**406 Taichung (TW)**

(74) Representative: **Lang, Christian
LangPatent Anwaltskanzlei
Ingolstädter Straße 5
80807 München (DE)**

(56) References cited:
**EP-A2- 0 201 905        WO-A1-2014/014815
DE-A1- 19 527 151      DE-A1-102015 122 709
JP-A- 2000 201 941     US-A1- 2005 288 674**

## Description

## CROSS-REFERENCE TO RELATED APPLICATIONS

This application claims priority to Taiwan Patent Application No. 104125361 filed August 5, 2015.

## BACKGROUND OF THE INVENTION

1. FIELD OF THE INVENTION

The embodiments disclosed herein relate to medical devices for use in repairing a weakened or fractured bone, and more particularly to internal bone fixation devices and methods for using the devices to repair a weakened or fractured bone.

2. DESCRIPTION OF RELATED ART

Fracture repair is the process of rejoining and realigning the ends of broken bones. Fracture repair is required when there is a need for restoration of the normal position and function of the broken bone. Throughout the stages of fracture healing, the bones must be held firmly in the correct position and supported until it is strong enough to bear weight. In the event the fracture is not properly repaired, malalignment of the bone may occur, resulting in possible physical dysfunction of the bone or joint of that region of the body

Currently there are several approaches to repair, strengthen and support a fractured bone. They include the use of bone fixation devices, such as wires, plates, rods, pins, nails, and screws to support the bone directly, and/or the addition of bone cement mixtures to a fractured bone. However, subsequent surgeries are required to remove these bone fixation devices after the fractured bones are healed.

Document DE 10 2015 122 709 A1 describes a conventional band for securing the bone. Document WO 2014 014 815 A1 describes a conventional bone fixation device for compressing a bone using a tension strip to surround the surface of the bone. Document EP 0 201 905 A2 discloses a self retaining permanently tensionable synthetic tie for use in surgery and orthopaedics. Document US 2005 288 674 A1 describes a bio-absorbable bone tie with a convex head which is an elongated band having a convex head portion and is used for securing fragments of a fractured bone together. Document JP 2000 201 941 A discloses a securing element for surgical use. The securing element has an elongated tie and a locking device adapted to engage with the tie to secure the tie and the locking device in a predetermined position. Document DE 19 527 151 A1 describes a device for bone fragment fixation. The device has a tension element, in the form of a flat band, which is formed into a loop or cylindrical shape.

Thus, there is a need in the art for an improved bone fixation device that repair, strengthen and support a fractured bone, the improved bone fixation device is easy to use, biodegradable, and causes minimal damage to the bone and supporting tissues.

## SUMMARY

Internal bone fixation devices and methods for using the devices to repair a weakened or fractured bone are disclosed herein. According to the present invention, there is provided a device for repairing a fractured bone as defined by claim 1. It includes a head portion having a conduit and a plurality of pawls formed therein along one side of the conduit; and an elongation strip having a hooked end and a plurality of ratchet teeth formed thereon along one side of the body of the strip. The head portion is connected to the non-hooked end of the elongation strip at an obtuse angle between 100-170 degrees, and the plurality of pawls of the head portion may engage the plurality of ratchet teeth when the elongation strip is inserted into the conduit of the head portion to form a loop around the weaken or facture bone.

According to some embodiments of the present disclosure, the head portion and the elongation strip of the bone fixation device are connected by forming a joined portion having an arc of 0.175 rad to 1.4 rad.

According to some embodiments of the present disclosure, the bone fixation device is composed by a bioabsorable material. Examples of bioabsorable material include, but are not limited to, alginate, biphasic calcum phosphate, calcium phosphate, calcium sulfate, chitosan, collagen, gelatin, hydroxyapatite (HA), polyalkylene esters, polyanhydrides, polyamide esters, polydioxanone, polyethylene glycol (PEG), polyactic acid (PLA) poly(lactic-co-glycolic acid) (PLGA), PLA-PLGA copolymer, poly(3-hydroxybutyrate-co-3-hydroxyvalerate), polyvinyl esters, polyvinyl alcohols, and tricalcium phosphate.

According to preferred embodiments of the present disclosure, the bone fixation device is an integral article.

The present bone fixation device may be used for the repair of bones that are weakened or fractured due to any of the bone diseases, which include, but are not limited to, osteoporosis, achondroplasia, bone cancer, fibrodysplasia ossificans progressiva, fibrous dysplasia, legg calve perthes disease, myeloma, osteogenesis imperfecta, osteomyelitis, osteopenia, osteoporosis, Paget's disease, scoliosis, and other similar diseases; or for the repair of bones that are weakened or fractured due to an injury, for example, a fall.

Examples of the weakened or fractured bone include, but are not limited to, metacarpal bone in the hand, the femur, tibia, fibula, humerus, ulna, radius, metatarsals, phalanx, phalanges, ribs, spine, vertebrae, clavicle and the like.

The bone fixation device may be used in a method for repairing a weaken or fractured bone that includes providing a device for use in repairing the weaken or fractured bone, the device comprises in its structure, a head portion having a conduit and a plurality of pawls formed therein along one side of the conduit; and an elongation strip having a hooked end and a plurality of ratchet teeth formed thereon along one side of the body of the strip. The head portion is connected to the non-hooked end of the elongation strip at an obtuse angle between 100-170 degrees, and the plurality of pawls of the head portion may engage the plurality of teeth when the elongation strip is inserted into the conduit of the head portion to form a loop around the weaken or facture bone.

Optionally, the head portion and the elongation strip of the bone fixation device are connected by forming a joined portion having an arc of 0.175 rad to 1.4 rad.

Optionally, the bone fixation device is composed by a bioabsorable material. Examples of bioabsorable material include, but are not limited to, alginate, biphasic calcum phosphate, calcium phosphate, calcium sulfate, chitosan, collagen, gelatin, hydroxyapatite (HA), polyalkylene esters, polyanhydrides, polyamide esters, polydioxanone, polyethylene glycol (PEG), polyactic acid (PLA) poly(lactic-co-glycolic acid) (PLGA), PLA-PLGA copolymer, poly(3-hydroxybutyrate-co-3-hydroxyvalerate), polyvinyl esters, polyvinyl alcohols, and tricalcium phosphate.

Preferably, the device is an integral article.

The details of one or more embodiments of this disclosure are set forth in the accompanying description below. Other features and advantages of the invention will be apparent from the detail descriptions, and from claims.

It is to be understood that both the foregoing general description and the following detailed description are by examples, and are intended to provide further explanation of the invention as claimed.

**BRIEF DESCRIPTION OF THE DRAWINGS**

The accompanying drawings, which are incorporated in and constitute a part of the specification, illustrate various example systems, methods and other exemplified embodiments of various aspects of the invention. The present description will be better understood from the following detailed description read in light of the accompanying drawings, where,

FIG 1 is a schematic drawing illustrating the bone fixation device 100 in accordance with one embodiment of the present disclosure;

FIG 2A is a schematic drawing depicting the bone fixation device 100 of FIG 1 forming a loop in accordance with one embodiment of the present disclosure;

FIG 2B is a schematic drawing depicting the loop of FIG 2A is tighten by engaging the plurality of teeth 122 with the plurality of pawls 154 in accordance with one embodiment of the present disclosure; and

FIG 3 is a schematic drawing illustrating the bone fixation device 300 in accordance with another embodiment of the present disclosure.

**DETAILED DESCRIPTIONOF THE PREFERRED EMBODIMENTS**

The detailed description provided below in connection with the appended drawings is intended as a description of the present disclosure and is not intended to represent the only forms in which the present disclosure may be constructed or utilized.

**1. Definition**

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The singular forms "a", "and", and "the" are used herein to include plural referents unless the context clearly dictates otherwise.

The term "host," "subject," or "patient" is used interchangeably herein and refers to a mammal, in which the present internal bone fixation device may be applied therein to stabilize any weaken or fractured bone. The term "mammal" refers to all members of the class Mammalia, including humans, primates, domestic and farm animals, such as rabbit, pig, sheep, and cattle; as well as zoo, sports or pet animals; and rodents, such as mouse and rat. Further, the term "host," "subject," or "patient" intended to refer to both the male and female gender unless one gender is specifically indicated. Accordingly, the term "host," "subject," or "patient" comprises any mammal which may benefit from the bone fixation device of the present disclosure. Examples of a "host," "subject," or "patient" include, but are not limited to, a human, rat, mouse, guinea pig, monkey, pig, goat, cow, horse, dog, cat, bird and fowl. In an exemplary embodiment, the patient is a human.

## 2. The present internal bone fixation device

Medical devices and methods for repairing weakened or fractured bones are disclosed herein. The devices disclosed herein act as internal bone fixation devices for stabilize internal weaken or fractured bones without the need of trocars or guide wires that commonly required when conventional bone fixation devices are employed. Further, the present internal bone fixation devices are biodegradable, thus eliminating the need of a subsequent surgery to remove the device from its host after the fractured bones are healed.

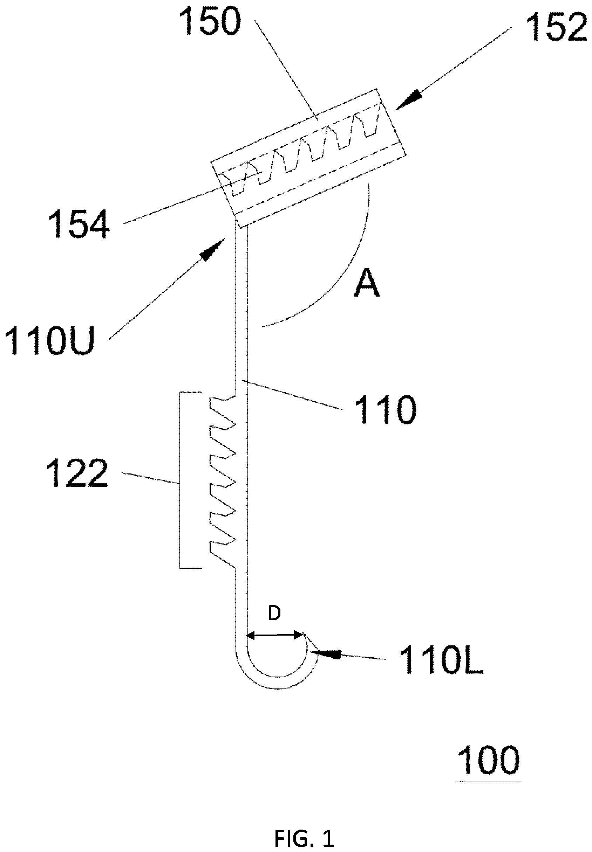

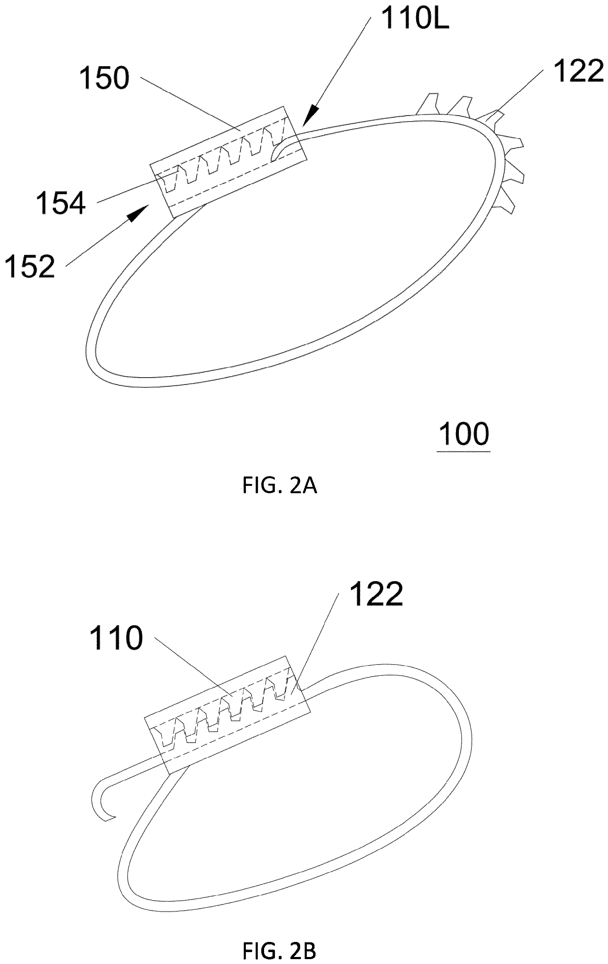

The main components of an embodiment of a device for repairing a weakened or fractured bone are depicted generally in **FIG** 1 in conjunction with **FIGs 2A** and **2B.** The bone fixation device **100** includes a head portion **150,** and an elongation strip **110** connected to the head portion **150** at an obtuse angle **(A).** The head portion **150** comprises in its structure, a conduit **152,** and a plurality of pawls **154** formed therein along one side of the conduit **152.** The elongation strip **110** has an elongation body having a hooked end **110L,** a non-hooked end **110U** connected to the head portion **150,** and a plurality of ratchet teeth **122** formed thereon along one side of the elongation body of the strip **110.** It is to be noted that the tip of the hooked end **110L** is sharpen enough to cut through muscles around a fractured bone, thus when the device **100** is in use, the elongation strip **110** can be inserted from the cut muscles and go around the fractured bone, then into the conduit **152** of the head portion **150** to form a loop around the fractured bone, the loop is then tighten by pulling the elongation strip **110** through the conduit **152** to engage the plurality of ratchet teeth **122** with the plurality of pawls **154,** thereby stabilizing the fractured bone (**FIGs 2A** and **2B**). Engagement of the ratchet teeth **122** and the pawls **154** also prevents reverse movement of the strip **110** through the conduit **152** of the head portion **150.** That way, the bone fixation device **100** is tighten around the weakened or fractured bone to hold the weakened or fractured bone in place.

According to embodiments of the present disclosure, the elongation strip **110** and the head portion **150** are connected at an obtuse angle (**A**) between 100-170 degrees, such as 100, 105, 110, 115, 120, 125, 130, 135, 140, 145, 150, 155, 160, 165, and 170 degrees; more preferably between 110-160 degrees, such as 110, 115, 120, 125, 130, 135, 140, 145, 150, 155, and 160 degrees. The obtuse angle between the elongation strip **110** and the head portion **150** allows the present bone fixation device **100** to comply with the shape or conformation of a bone surface when stabilizing a fractured bone, so that when the loop is tighten, such as by pulling the elongation strip **110** through the conduit **152** to engage the plurality of ratchet teeth **122** with the plurality of pawls **154,** the stress generated therein can be distributed evenly to prevent the elongation strip **110** from being broken.

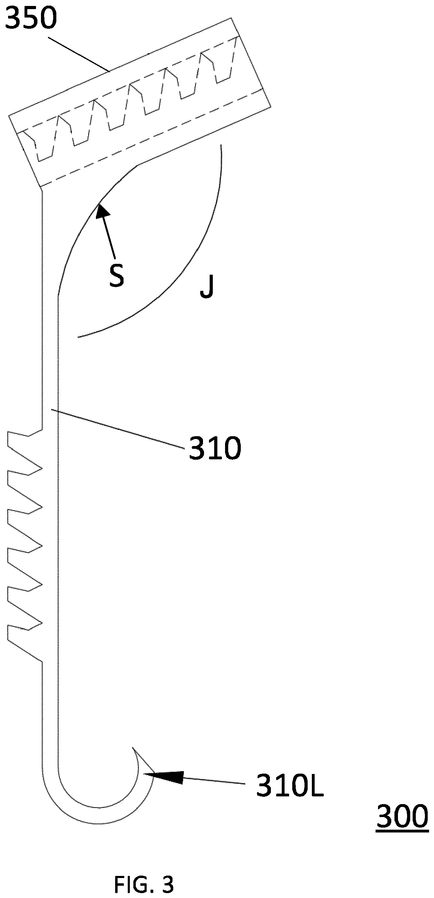

**FIG 3** depicts another embodiment of the present bone fixation device **300,** in which the structure of the device **300** is relatively the same with the device **100** described above in **FIGs 1** and **2,** except the connected portion between the elongation strip **310** and the head portion **350.** In this embodiment, the elongation strip **310** and the head portion **350** are connected by forming a joined portion (**J**) having an arc of 0.175 to 1.4 rad (radian) at an obtuse angle (**A**) as defined above. The term "radian" refers to a unit for measuring angles. One radian is the angle made at the center of a circle by an arc whose length is equal to the radius of the circle, and is represented by the equation:

$$rad = \frac{\pi}{100} \times \deg$$

By such design (i.e., the elongation strip **310** and the head portion **350** are connected by forming the joined potion (**J**) having an arc of 0.175 to 1.4 rad), the stress generated while stabilizing a fractured bone can be evenly distributed.

The present bone fixation device **100, 300** is preferably made by a material that is compatible and absorbable by its host (*e.g.*, a human), such that the present device may be left inside the host after a fractured bone is stabilized, thereby eliminates the need of a subsequent surgery to remove the device therefrom. Examples of such material include, but are not limited to, alginate, biphasic calcum phosphate, calcium phosphate, calcium sulfate, chitosan, collagen, gelatin, hydroxyapatite (HA), polyalkylene esters, polyanhydrides, polyamide esters, polydioxanone, polyethylene glycol (PEG), polyactic acid (PLA) poly(lactic-co-glycolic acid) (PLGA), PLA-PLGA copolymer, poly(3-hydroxybutyrate-co-3-hydroxyvalerate), polyvinyl esters, polyvinyl alcohols, and tricalcium phosphate.

According to preferred embodiments of the present disclosure, the preset bone fixation device is formed into an integral article.

It should be noted that the present bone fixation device **100, 300** may come in various sizes and dimensions, and a physician may chose a suitable size of the present device according to the size and location of the weakened or fractured bone needs to be stabilized. According to some embodiments of the present disclosure, the head portion **150, 350** of has a length of about 0.3-6.5 cm, such as 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4 and 6.5 cm. The elongation strip **110, 310** has a thickness of about 0.2-1 cm, such as 0.2, 0.3, 0.4, 0.5, 0.6, 0.7, 0.8, 0.9, and 1 cm, preferably about 0.2-0.5 cm, such as 0.2, 0.3, 0.4, and 0.5 cm; and a width of about 0.2-5 cm, such as 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 and 5 cm, preferably about 0.5-3 cm, such as 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0 cm; and a length of about 3-15 cm such as 3, 4, 5, 6, 7, 8, 9, 10, 11, 12, 13, 14, and 15 cm, preferably about 5-12 cm, such as 5, 6, 7, 8, 9, 10, 11, and 12 cm.

According to further embodiments of the present disclosure, the non-hooked end **110L, 310L** of the elongation strip **110, 310** has a shape of a circle of 2/10 - 9/10, such as a circle of 2/10, 3/10, 4/10, 5/10, 6/10, 7/10, 8/10 and 9/10; further, the tip of non-hooked end **110L, 310L** is spaced apart from the elongation body by a distance (D) of about 0.5-7 cm, such as 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9 and 7.0 cm.

The bone fixation device disclosed herein may be used for the repair of bones that are weakened or fractured due to any of the bone diseases, which include, but are not limited to, osteoporosis, achondroplasia, bone cancer, fibrodysplasia ossificans progressiva, fibrous dysplasia, legg calve perthes disease, myeloma, osteogenesis imperfecta, osteomyelitis, osteopenia, osteoporosis, Paget's disease, scoliosis, and other similar diseases. The bone fixation device disclosed herein may also be used for the repair of bones that are weakened or fractured due to an injury, for example, a fall.

Examples of the weakened or fractured bone include, but are not limited to, metacarpal bone in the hand, the femur, tibia, fibula, humerus, ulna, radius, metatarsals, phalanx, phalanges, ribs, spine, vertebrae, clavicle and other bones, which are still within the scope and spirit of the disclosed embodiments.

It will be understood that the above description of embodiments is given by way of example only and that various modifications may be made by those with ordinary skill in the art. The above specification, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the scope of the invention, which is defined by the appended claims.

## Claims

1. A bone fixation device (100) for stabilizing a weakened or fractured bone, comprising:

   a head portion (150) having a conduit (152) and a plurality of pawls (154) formed therein along one side of the conduit (152); and
   an elongation strip (110) having a plurality of ratchet teeth (122) formed thereon along one side of the body of the elongation strip (110);
   wherein the plurality of pawls (154) of the head portion (150) engage the plurality of ratchet teeth (122) when the elongation strip (100) is inserted into the head portion (150) to form a loop around the weakened or fractured bone, so as to stabilize the weakened or fractured bone,
   **characterized in that**
   the elongation strip (110) further has a hooked end (110L),
   and **in that**
   the head portion (150) is connected to the non-hooked end (110U) of the elongation strip (110) at an obtuse angle between 100-170 degrees, the obtuse angle allowing the bone fixation device (100) to comply with the shape or conformation of a bone surface when stabilizing the weakened or fractured bone, so that when the loop is tightened, the stress generated therein can be distributed evenly to prevent the elongation strip (110) from being broken.

2. The bone fixation device of claim 1, wherein the head portion (150) and the elongation strip (110) are connected by forming a joined portion having an arc of 0.175 rad to 1.4 rad.

3. The bone fixation device of claim 1, wherein the device is composed by a bioabsorbable material.

4. The bone fixation device of claim 3, wherein the bioabsorbable material is selected from the group consisting of, alginate, biphasic calcium phosphate. calcium phosphate, calcium sulfate, chitosan, collagen, gelatin, hydroxyapatite (HA), polyalkylene esters, polyanhydrides, polyamide esters, polydioxanone, polyethylene glycol (PEG), polyactic acid (PLA) poly(lactic-co-glycolic acid) (PLGA), PLA-PLGA copolymer, poly(3-hydroxybutyrate-co-3-hydroxyvalerate), polyvinyl esters, polyvinyl alcohols, and tricalcium phosphate.

5. The bone fixation device of claim 1, wherein the device is an integral article.

## Patentansprüche

1. Knochenfixationsvorrichtung (100) zur Stabilisierung eines geschwächten oder gebrochenen Knochens, umfassend:

einen Kopfabschnitt (150), der einen Kanal (152) und eine Mehrzahl von Sperrklinken (154) aufweist, die darin entlang einer Seite des Kanals (152) ausgebildet sind, und

einen Verlängerungsstreifen (110), der eine Mehrzahl von Sperrzähnen (122) aufweist, die daran entlang einer Seite des Körpers des Verlängerungsstreifens (110) ausgebildet sind,

wobei die Vielzahl von Sperrklinken (154) des Kopfabschnitts (150) mit der Vielzahl von Sperrzähnen (122) in Eingriff kommen, wenn der Verlängerungsstreifen (100) in den Kopfabschnitt (150) eingesteckt wird, um eine Schlaufe um den geschwächten oder gebrochenen Knochen herum zu bilden, um den geschwächten oder gebrochenen Knochen zu stabilisieren, **dadurch gekennzeichnet, dass**

der Verlängerungsstreifen (110) ferner ein Hakenende (110L) aufweist,

und dass

der Kopfabschnitt (150) mit dem keinen Haken aufweisenden Ende (110U) des Verlängerungsstreifens (110) in einem stumpfen Winkel zwischen 100 bis 170 Grad verbunden ist, wobei es der stumpfe Winkel der Knochenfixationsvorrichtung (100) ermöglicht, sich nach der Form oder Konformation einer Knochenoberfläche zu richten, wenn der geschwächte oder gebrochene Knochen stabilisiert wird, sodass, wenn die Schlaufe festgezogen wird, die darin erzeugte Spannung gleichmäßig verteilt werden kann, wodurch verhindert wird, dass der Verlängerungsstreifen (110) bricht.

2. Knochenfixationsvorrichtung nach Anspruch 1, bei welcher der Kopfabschnitt (150) und der Verlängerungsstreifen (110) durch Bildung eines Verbindungsabschnitts, der einen Bogen von 0,175 rad bis 1,4 rad aufweist, verbunden sind.

3. Knochenfixationsvorrichtung nach Anspruch 1, wobei sich die Vorrichtung aus einem bioabsorbierbaren Material zusammensetzt.

4. Knochenfixationsvorrichtung nach Anspruch 3, bei welcher das bioabsorbierbare Material aus einer Gruppe ausgewählt ist, die Alginat, zweiphasiges Calciumphosphat, Calciumphosphat, Calciumsulfat, Chitosan, Kollagen, Gelatine, Hydroxylapatit (HA), Polyalkylenester, Polyanhydride, Polyamidester, Polydioxanon, Polyethylenglykol (PEG), Polymilchsäure (PLA), Poly(milchsäure-co-glykolsäure) (PLGA), PLA-PLGA-Copolymer, Poly-(3-hydroxybutyrat-co-3-hydroxyvalerat), Polyvinylester, Polyvinylalkohole und Tricalciumphosphat umfasst.

5. Knochenfixationsvorrichtung nach Anspruch 1, wobei die Vorrichtung ein integraler Gegenstand ist.

**Revendications**

1. Dispositif de fixation d'os (100) pour stabiliser un os affaibli ou fracturé, comprenant:

une partie tête (150) ayant un conduit (152) et une pluralité de cliquets (154) formés à l'intérieur de celui-ci sur un côté du conduit (152); et une bande d'allongement (110) ayant des dents d'encliquetage (122) formées sur celle-ci le long d'un côté du corps de la bande d'allongement (110);

dans lequel la pluralité de cliquets (154) de la partie tête (150) se mettent en prise avec la pluralité de dents d'encliquetage (122) lorsque la bande d'allongement (100) est insérée dans la partie tête (150) pour former une boucle autour de l'os affaibli ou fracturé, de manière à stabiliser l'os affaibli ou fracturé **caractérisé en ce que** la bande d'allongement (110) présente en outre une extrémité à crochet (110L),

et **en ce que**

la partie tête (150) est raccordée à l'extrémité sans crochet (110U) de la bande d'allongement (110) au niveau d'un angle obtus entre 100 et 170 degrés, l'angle obtus permettant au dispositif de fixation d'os (100) de se conformer à la forme ou à la conformation d'une surface osseuse lors de la stabilisation de l'os fracturé ou affaibli, de sorte que lorsque la boucle est serrée, la contrainte généré à l'intérieur de celle-ci puisse être distribuée de façon homogène pour empêcher la rupture de la bande d'allongement (110).

2. Dispositif de fixation d'os selon la revendication 1, dans lequel la partie tête (150) et la bande d'allongement (110) sont raccordées par la formation d'une partie jointe ayant un arc de 0,175 à 1,4 rad.

3. Dispositif de fixation d'os selon la revendication 1, dans lequel le dispositif est composé d'un matériau bioabsorbable.

4. Dispositif de fixation d'os selon la revendication 3, dans lequel le matériau bioabsorbable est sélectionné dans le groupe constitué de l'alginate, du phosphate de calcium biphasique, du phosphate de calcium, du sulfate de calcium, du chitosane, du collagène, de la gélatine, de l'hydroxyapatite (HA), des esters de polyalkylène, des polyanhydrides, des esters de polyamide, du polydioxanone, du polyéthylène glycol (PEG), de l'acide polylactique (PLA) poly(acide lactique-co-glycolique) (PLGA), d'un copolymère PLA-PLGA, du poly(3-hydroxybutyrate-co-3-hydroxyvalérate), des esters de polyvinyle, des alcools polyvinyliques, et du phosphate tricalcique.

**5.** Dispositif de fixation d'os selon la revendication 1, dans lequel le dispositif de fixation d'os est un article intégral.

FIG. 1

FIG. 2A

FIG. 2B

350

S          J

310

310L

300

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 104125361
- DE 102015122709 A1
- WO 2014014815 A1
- EP 0201905 A2
- US 2005288674 A1
- JP 2000201941 A
- DE 19527151 A1